# EUROPÄISCHE PATENTSCHRIFT

(11) **EP 2 734 631 B1**
(45) Veröffentlichungstag und Bekanntmachung des Hinweises auf die Patenterteilung: **31.08.2016**
(21) Anmeldenummer: 12735553.5
(22) Anmeldetag: 17.07.2012
(51) Int. Cl.: C12P 13/00

(54) **OXIDATION UND AMINIERUNG VON PRIMÄREN ALKOHOLEN**
OXIDATION AND AMINATION OF SECONDARY ALCOHOLS
OXYDATION ET AMINATION D'ALCOOLS SECONDAIRES

(30) Priorität: 20.07.2011 EP 11174729; 05.08.2011 EP 11006458; 09.01.2012 EP 12150451
(43) Veröffentlichungstag der Anmeldung: 28.05.2014
(73) Patentinhaber: Evonik Degussa GmbH, 45128 Essen (DE)
(72) Erfinder: PÖTTER, Markus, 201702 Shangai (CN); HAAS, Thomas, 48161 Münster (DE); PFEFFER, Jan, Christoph, 63452 Hanau (DE); SKERRA, Arne, 85354 Freising (DE); KROUTIL, Wolfgang, A-8042 Graz (AT); LERCHNER, Alexandra, 85375 Neufahrn bei Freising (DE); SATTLER, Johann, H., A-8990 Bad Aussee (AT); SCHAFFER, Steffen, 45699 Herten (DE); TAUBER, Katharina, Christin, A-9431 Wolfsberg (AT)
(86) Internationale Anmeldenummer: PCT/EP2012/063994
(87) Internationale Veröffentlichungsnummer: WO 2013/011018

(56) Entgegenhaltungen:
- EP-A1- 2 022 852
- WO-A2-2010/085731
- DE-A1-102007 060 705
- YUN H ET AL: "Simultaneous synthesis of enantiomerically pure (R)-1-phenylethanol and (R)-alpha-methylbenzylamine from racemic alpha-methylbenzylamine using omega-transaminase/alcohol dehydrogenase/glucose dehydrogenase coupling reaction", BIOTECHNOLOGY LETTERS, KEW, SURREY, GB, Bd. 25, Nr. 10, 1. Mai 2003 (2003-05-01), Seiten 809-814, XP002420353, ISSN: 0141-5492, DOI: 10.1023/A:1023500406897
- MATSUYAMA A ET AL: "Practical application of recombinant whole-cell biocatalysts for the manufacturing of pharmaceutical intermediates such as chiral alcohols", ORGANIC PROCESS RESEARCH AND DEVELOPMENT, CAMBRIDGE, GB, Bd. 6, 1. Januar 2002 (2002-01-01), Seiten 558-561, XP002344756, DOI: 10.1021/OP025514S
- WECKBECKER A ET AL: "Improved synthesis of chiral alcohols with Escherichia coli cells co-expressing pyridine nucleotide transhydrogenase, NADP(+)-dependent alcohol dehydrogenase and NAD(+)-dependent formate dehydrogenase", BIOTECHNOLOGY LETTERS, KEW, SURREY, GB, Bd. 26, 1. November 2004 (2004-11-01), Seiten 1739-1744, XP002344979, ISSN: 0141-5492, DOI: 10.1007/S10529-004-3746-2
- YUN HYUNGDON ET AL: "Asymmetric Synthesis of (S)-alpha-Methylbenzylamine by Recombinant Escherichia coli Co-Expressing Omega-Transaminase and Acetolactate Synthase", BIOSCIENCE BIOTECHNOLOGY AND BIOCHEMISTRY, Bd. 72, Nr. 11, November 2008 (2008-11), Seiten 3030-3033, XP002665945, ISSN: 0916-8451
- KOSZELEWSKI, DOMINIK ET AL: "Synthesis of Optically Active Amines Employing Recombinant .omega.-Transaminases in E. coli Cells", CHEMCATCHEM , 2(1), 73-77 CODEN: CHEMK3; ISSN: 1867-3880, 2010, XP002665946,

### SEQUENCE LISTING

<110> Evonik Degussa GmbH
<120> Oxidation von Alkoholen
<130> 2011E00296DE
<160> "=
<170> PatentIn version 3.5
<210> 1
   <211> 57
   <212> PRT
   <213> artificial
<220>
   <223> Part of sequence
<400> 1
   Val Val Ala Ala Arg Trp Leu Glu Glu Lys Ile Leu Glu Ile Gly Ala
   1               5                   10                  15
   Asp Lys Val Ala Ala Phe Val Gly Glu Pro Ile Gln Gly Ala Gly Gly
               20                  25                  30
   Val Ile Val Pro Pro Ala Thr Tyr Trp Pro Glu Ile Glu Arg Ile Cys
           35                  40                  45
   Arg Lys Tyr Asp Val Leu Leu Val Ala
       50                  55
<210> 2
   <211> 57
   <212> PRT
   <213> artificial
<220>
   <223> part of sequence
<400> 2
   Ala His Cys Val Ala Glu Leu Glu Ala Leu Ile Glu Arg Glu Gly Ala
   1               5                   10                  15
   Asp Thr Ile Ala Ala Phe Ile Gly Glu Pro Ile Leu Gly Thr Gly Gly
               20                  25                  30
   Ile Val Pro Pro Pro Ala Gly Tyr Trp Glu Ala Ile Gln Thr Val Leu
           35                  40                  45
   Asn Lys His Asp Ile Leu Leu Val Ala
       50                  55
<210> 3
   <211> 57
   <212> PRT
   <213> artificial
<220>
   <223> Part of sequence
<400> 3
   Gln His Cys Ala Asp Lys Leu Glu Glu Met Ile Leu Ala Glu Gly Pro
   1               5                   10                  15
   Glu Thr Ile Ala Ala Phe Ile Gly Glu Pro Ile Leu Gly Thr Gly Gly
               20                  25                  30
   Ile Val Pro Pro Pro Ala Gly Tyr Trp Glu Lys Ile Gln Ala Val Leu
           35                  40                  45
   Lys Lys Tyr Asp Val Leu Leu Val Ala
       50                  55
<210> 4
   <211> 57
   <212> PRT
   <213> artificial
<220>
   <223> part of sequence
<400> 4
   Asp Asp Leu Val Gln Glu Phe Glu Asp Arg Ile Glu Ser Leu Gly Pro
   1               5                   10                  15
   Asp Thr Ile Ala Ala Phe Leu Ala Glu Pro Ile Leu Ala Ser Gly Gly
               20                  25                  30
   Val Ile Ile Pro Pro Ala Gly Tyr His Ala Arg Phe Lys Ala Ile Cys
           35                  40                  45
   Glu Lys His Asp Ile Leu Tyr Ile Ser
       50                  55
<210> 5
   <211> 57
   <212> PRT
   <213> artificial
<220>
   <223> part of sequence
<400> 5
   Ala Glu Leu Ala Asn Glu Leu Glu Arg Ile Val Ala Leu His Asp Ala
   1               5                   10                  15
   Ser Thr Ile Ala Ala Val Ile Val Glu Pro Val Ala Gly Ser Thr Gly
               20                  25                  30
   Val Ile Leu Pro Pro Lys Gly Tyr Leu Gln Lys Leu Arg Glu Ile Cys
           35                  40                  45
   Thr Lys His Gly Ile Leu Leu Ile Phe
       50                  55
<210> 6
   <211> 57
   <212> PRT
   <213> artificial
<220>
   <223> part of sequence
<400> 6
   Ala Glu Leu Ala Asn Glu Leu Glu Arg Ile Val Ala Leu His Asp Ala
   1               5                   10                  15
   Ser Thr Ile Ala Ala Val Ile Val Glu Pro Val Ala Gly Ser Thr Gly
               20                  25                  30
   Val Ile Leu Pro Pro Lys Gly Tyr Leu Gln Lys Leu Arg Glu Ile Cys
           35                  40                  45
   Thr Lys His Gly Ile Leu Leu Ile Phe
       50                  55
<210> 7
   <211> 57
   <212> PRT
   <213> artificial
<220>
   <223> part of sequence
<400> 7
   Ala His Leu Ala Asp Glu Leu Glu Arg Ile Ile Ala Leu His Asp Ala
   1               5                   10                  15
   Ser Thr Ile Ala Ala Val Ile Val Glu Pro Met Ala Gly Ser Thr Gly
               20                  25                  30
   Val Leu Val Pro Pro Lys Gly Tyr Leu Glu Lys Leu Arg Glu Ile Thr
           35                  40                  45
   Ala Arg His Gly Ile Leu Leu Ile Phe
       50                  55
<210> 8
   <211> 57
   <212> PRT
   <213> artificial
<220>
   <223> part of sequence
<400> 8
   Ala His Leu Ala Asp Glu Leu Glu Arg Ile Val Ala Leu His Asp Pro
   1               5                   10                  15
   Ser Thr Ile Ala Ala Val Ile Val Glu Pro Leu Ala Gly Ser Ala Gly
               20                  25                  30
   Val Leu Val Pro Pro Val Gly Tyr Leu Asp Lys Leu Arg Glu Ile Thr
           35                  40                  45
   Thr Lys His Gly Ile Leu Leu Ile Phe
       50                  55
<210> 9
   <211> 57
   <212> PRT
   <213> artificial
<220>
   <223> part of sequence
<400> 9
   Val Glu Leu Ala Asn Glu Leu Leu Lys Leu Ile Glu Leu His Asp Ala
   1               5                   10                  15
   Ser Asn Ile Ala Ala Val Ile Val Glu Pro Met Ser Gly Ser Ala Gly
               20                  25                  30
   Val Leu Val Pro Pro Val Gly Tyr Leu Gln Arg Leu Arg Glu Ile Cys
           35                  40                  45
   Asp Gln His Asn Ile Leu Leu Ile Phe
       50                  55
<210> 10
   <211> 57
   <212> PRT
   <213> artificial
<220> Page 5 of 12
   <223> part of sequence
<400> 10
   Ile Ala Leu Ala Asp Glu Leu Leu Lys Leu Ile Glu Leu His Asp Ala
   1               5                   10                  15
   Ser Asn Ile Ala Ala Val Phe Val Glu Pro Leu Ala Gly Ser Ala Gly
               20                  25                  30
   Val Leu Val Pro Pro Glu Gly Tyr Leu Lys Arg Asn Arg Glu Ile Cys
           35                  40                  45
   Asn Gln His Asn Ile Leu Leu Val Phe
       50                  55
<210> 11
   <211> 57
   <212> PRT
   <213> artificial
<220>
   <223> part of sequence
<400> 11
   Pro Ala Tyr Ser Ala Ala Phe Glu Ala Gln Leu Ala Gln His Ala Gly
   1               5                   10                  15
   Glu Leu Ala Ala Val Val Val Glu Pro Val Val Gln Gly Ala Gly Gly
               20                  25                  30
   Met Arg Phe His Asp Pro Arg Tyr Leu His Asp Leu Arg Asp Ile Cys
           35                  40                  45
   Arg Arg Tyr Glu Val Leu Leu Ile Phe
       50                  55
<210> 12
   <211> 57
   <212> PRT
   <213> artificial
<220>
   <223> part of sequence
<400> 12
   Glu Arg Asp Met Val Gly Phe Ala Arg Leu Met Ala Ala His Arg His
   1               5                   10                  15
   Glu Ile Ala Ala Val Ile Ile Glu Pro Ile Val Gln Gly Ala Gly Gly
               20                  25                  30
   Met Arg Met Tyr His Pro Glu Trp Leu Lys Arg Ile Arg Lys Ile Cys
   35 40 45
   Asp Arg Glu Gly Ile Leu Leu Ile Ala
   50 55
<210> 13
   <211> 57
   <212> PRT
   <213> artificial
<220>
   <223> part of sequence
<400> 13
   Asp Gln Cys Leu Arg Glu Leu Ala Gln Leu Leu Glu Glu His His Glu
   1 5 10 15
   Glu Ile Ala Ala Leu Ser Ile Glu Ser Met Val Gln Gly Ala Ser Gly
   20 25 30
   Met Ile Val Met Pro Glu Gly Tyr Leu Ala Gly Val Arg Glu Leu Cys
   35 40 45
   Thr Thr Tyr Asp Val Leu Met Ile Val
   50 55
<210> 14
   <211> 54
   <212> PRT
   <213> artificial
<220>
   <223> part of sequence
<400> 14
   Ala Asn Glu Ile Asp Arg Ile Met Thr Trp Glu Leu Ser Glu Thr Ile
   1 5 10 15
   Ala Gly Val Ile Met Glu Pro Ile Ile Thr Gly Gly Gly Ile Leu Met
   20 25 30
   Pro Pro Asp Gly Tyr Met Lys Lys Val Glu Asp Ile Cys Arg Arg His
   35 40 45
   Gly Ala Leu Leu Ile Cys
   50
<210> 15
   <211> 56
   <212> PRT
   <213> artificial
<220>
   <223> part of sequence
<400> 15
   Leu Leu Ser Val Lys Tyr Thr Arg Arg Met Ile Glu Asn Tyr Gly Pro
   1               5                   10                  15
   Glu Gln Val Ala Ala Val Ile Thr Glu Val Ser Gln Gly Ala Gly Ser
               20                  25                  30
   Ala Met Pro Pro Tyr Glu Tyr Ile Pro Gln Phe Arg Lys Met Thr Lys
           35                  40                  45
   Glu Leu Gly Val Leu Trp Ile Asn
       50                  55
<210> 16
   <211> 56
   <212> PRT
   <213> artificial
<220>
   <223> part of sequence
<400> 16
   Leu Leu Ser Val Lys Tyr Thr Arg Arg Met Ile Glu Asn Tyr Gly Pro
   1               5                   10                  15
   Glu Gln Val Ala Ala Val Ile Thr Glu Val Ser Gln Gly Ala Gly Ser
               20                  25                  30
   Ala Met Pro Pro Tyr Glu Tyr Ile Pro Gln Ile Arg Lys Met Thr Lys
           35                  40                  45
   Glu Leu Gly Val Leu Trp Ile Asn
       50                  55
<210> 17
   <211> 56
   <212> PRT
   <213> artificial
<220>
   <223> part of sequence
<400> 17
   Leu Leu Ser Val Lys Tyr Thr Arg Arg Met Ile Glu Asn Tyr Gly Pro
   1               5                   10                  15
   Glu Gln Val Ala Ala Val Ile Thr Glu Val Ser Gln Gly Val Gly Ser
               20                  25                  30
   Thr Met Pro Pro Tyr Glu Tyr Val Pro Gln Ile Arg Lys Met Thr Lys
           35                  40                  45
   Glu Leu Gly Val Leu Trp Ile Ser
       50                  55
<210> 18
   <211> 56
   <212> PRT
   <213> artificial
<220>
   <223> part of sequence
<400> 18
   Lys Tyr Ala Ser Asp Val His Asp Leu Ile Gln Phe Gly Thr Ser Gly
   1               5                   10                  15
   Gln Val Ala Gly Phe Ile Gly Glu Ser Ile Gln Gly Val Gly Gly Ile
               20                  25                  30
   Val Glu Leu Ala Pro Gly Tyr Leu Pro Ala Ala Tyr Asp Ile Val Arg
           35                  40                  45
   Lys Ala Gly Gly Val Cys Ile Ala
       50                  55
<210> 19
   <211> 57
   <212> PRT
   <213> artificial
<220>
   <223> part of sequence
<400> 19
   Leu Ala Glu Leu Asp Tyr Ala Phe Asp Leu Ile Asp Arg Gln Ser Ser
   1               5                   10                  15
   Gly Asn Leu Ala Ala Phe Ile Ala Glu Pro Ile Leu Ser Ser Gly Gly
               20                  25                  30
   Ile Ile Glu Leu Pro Asp Gly Tyr Met Ala Ala Leu Lys Arg Lys Cys
           35                  40                  45
   Glu Ala Arg Gly Met Leu Leu Ile Leu
       50                  55
<210> 20
   <211> 6949
   <212> DNA
   <213> artificial
<220>
   <223> plasmid ptZE03_aLKj
<400> 1

## Beschreibung

Die vorliegende Erfindung betrifft ein Verfahren zur Oxidation von Alkoholen umfassend die Schritte
a) Bereitstellen eines primären Alkohols der Formel

   HO-(CH₂)ₓ-R¹,

   wobei R¹ aus der Gruppe ausgewählt ist, die -OH, -SH, -NH₂ und -COOR² umfasst, x wenigstens 3 ist und R² aus der Gruppe ausgewählt ist, die H, Alkyl und Aryl umfasst,
b) Oxidation des primären Alkohols durch Kontaktieren mit einer NAD(P)⁺-abhängigen Alkoholdehydrogenase, und
c) Kontaktieren des Oxidationsproduktes aus Schritt a) mit einer Transaminase,
wobei es sich bei der NAD(P)⁺-abhängigen Alkoholdehydrogenase und/oder der Transaminase um ein rekombinantes oder isoliertes Enzym handelt,
und die Verwendung eines derartigen Ganzzellkatalysators zur Oxidation eines primären Alkohols.

Polyamide sind eine Klasse von Polymeren, die durch sich wiederholende Amidgruppen gekennzeichnet sind. Der Begriff "Polyamide" betrifft im Unterschied zu den chemisch verwandten Proteinen gewöhnlich synthetische, im Handel erhältliche, thermoplastische Kunststoffe. Polyamide werden von primären Aminen oder von sekundären Aminen abgeleitet, die herkömmlich beim Cracken von Kohlenwasserstoffen gewonnen werden. Es können jedoch auch Derivate, genauer Aminocarbonsäure, Lactame und Diamine, zur Polymerherstellung verwendet werden. Von Interesse sind weiterhin kurzkettige, gasförmige Alkane als Edukte, die ausgehend von nachwachsenden Rohstoffen mit biotechnologischen Verfahren gewonnen werden können.

Viele kommerziell stark nachgefragte Polyamide werden ausgehend von Lactamen hergestellt. Zum Beispiel kann man "Polyamid 6" durch Polymerisation von ε-Caprolactam und "Polyamid 12" durch Polymerisierung von Laurinlactam erhalten. Weitere kommerziell interessante Produkte umfassen Copolymere von Lactam, z. B. Copolymere von ε-Caprolactam und Laurinlactam.

Die konventionelle chemisch-technische Erzeugung von Aminen ist von der Versorgung mit fossilen Rohstoffen abhängig, ineffizient, und dabei fallen große Mengen unerwünschter Nebenprodukte an, in manchen Schritten der Synthese bis zu 80 %. Ein Beispiel für einen solchen Prozess stellt die Herstellung von Laurinlactam dar, das konventionell durch Trimerisierung von Butadien gewonnen wird. Das Trimerisierungsprodukt Cyclododekatrien wird hydriert und das daraus resultierende Cyclododekan wird zu Cyclodekanon oxidiert, das anschließend mit Hydroxylamin zu Cyclododekanoxin umgesetzt wird, welche schließlich über eine Beckmann-Umlagerung zu Laurinlactam umgewandelt wird.

Eingedenk dieser Nachteile wurden Verfahren entwickelt, um Amine unter Verwendung von Biokatalysatoren ausgehend von nachwachsenden Rohstoffen zu gewinnen. PCT/EP 2008/067447 beschreibt ein biologisches System zur Herstellung chemisch verwandter Produkte, genauer ω-Aminocarbonsäuren, unter Verwendung einer Zelle, die eine Reihe von geeigneten enzymatischen Aktivitäten aufweist und in der Lage ist, Carbonsäuren zu entsprechender ω-Aminocarbonsäure umzuwandeln. Ein bekannter Nachteil des dabei verwendeten AlkBGT-Oxidasesystems aus *Pseudomonas putida* GPO1 besteht jedoch darin, dass es nicht in der Lage ist, eine selektive Oxidation von aliphatischen Alkanen zu primären Alkoholen zu leisten. Vielmehr treten eine Vielzahl von Oxidationsprodukten auf; insbesondere erhöht sich der Anteil an höher oxidierten Produkten wie dem entsprechenden Aldehyd, Keton oder der entsprechenden Carbonsäure mit zunehmender Reaktionsdauer (C. Grant, J. M. Woodley and F. Baganz (2011), Enzyme and Microbial Technology 48, 480-486), was die Ausbeute an erwünschtem Amin entsprechend verringert.

Vor diesem Hintergrund besteht die der Erfindung zu Grunde liegende Aufgabe darin, ein verbessertes Verfahren zur Oxidation von Alkoholen unter Verwendung von Biokatalysatoren bereitzustellen. Eine weitere Aufgabe besteht darin, das Verfahren dahingehend zu verbessern, dass die Ausbeute erhöht und/oder die Konzentration an Nebenprodukten gesenkt wird. Schließlich besteht Bedarf an einem Verfahren, dass die Herstellung von Polyamiden oder Edukten zu deren Herstellung auf der Basis von nachwachsenden Rohstoffen gestattet.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Erfindungsgemäß wird die Aufgabe in einem ersten Aspekt gelöst durch ein Verfahren zur Oxidation von Alkoholen umfassend die Schritte
a) Bereitstellen eines primären Alkohols der Formel

   HO-(CH₂)ₓ-R¹,

   wobei R¹ aus der Gruppe ausgewählt ist, die -OH, -SH, -NH₂ und -COOR² umfasst, x wenigstens 3 ist und R² aus der Gruppe ausgewählt ist, die H, Alkyl und Aryl umfasst,
b) Oxidation des primären Alkohols durch Kontaktieren mit einer NAD(P)⁺-abhängigen Alkoholdehydrogenase, und
c) Kontaktieren des Oxidationsproduktes aus Schritt a) mit einer Transaminase,
   wobei es sich bei der NAD(P)⁺- Alkoholdehydrogenase und/oder der Transaminase um ein rekombinantes oder isoliertes Enzym handelt.

In einer ersten Ausführungsform des ersten Aspektes erfolgt Schritt a) durch Hydroxylierung eines Alkans der Formel

H-(CH₂)ₓ - R¹

durch eine Monooxygenase, die bevorzugt rekombinant oder isoliert ist.

In einer zweiten Ausführungsform des ersten Aspektes, die auch eine Ausführungsform der ersten Ausführungsform darstellt, handelt es sich bei der NAD(P)⁺-abhängigen

Alkoholdehydrogenase um eine NAD(P)⁺-abhängige Alkoholdehydrogenase mit wenigstens einem Zinkatom als Cofaktor.

In einer dritten Ausführungsform des ersten Aspektes, die eine Ausführungsform der zweiten Ausführungsform darstellt, handelt es sich bei der Alkoholdehydrogenase um die Alkoholdehydrogenase von *Bacillus stearothermophilus* (Datenbankcode P42328) oder eine Variante davon.

In einer vierten Ausführungsform des ersten Aspektes, die eine Ausführungsform der ersten bis dritten Ausführungsform darstellt, ist die Monooxygenase aus der Gruppe ausgewählt, die AlkBGT von *Pseudomonas putida,* Cytochrom P450 aus *Candida tropicalis* oder aus *Cicer arietinum* umfasst.

In einer fünften Ausführungsform des ersten Aspektes, die auch eine Ausführungsform der ersten bis vierten Ausführungsform darstellt, ist die Transaminase aus der Gruppe von Transaminasen und deren Varianten ausgewählt, die dadurch gekennzeichnet sind, dass sie an der Position der Aminosäuresequenz, die Val224 aus der Transaminase von *Chromobacterium violaceum* ATCC 12472 (Datenbankcode NP_901695) entspricht, eine Aminosäure ausgewählt aus der Gruppe umfassend Isoleucin, Valin, Phenylalanin, Methionin und Leucin aufweist, und an der Position der Aminosäuresequenz, die Gly230 aus der Transaminase von *Chromobacterium violaceum* ATCC 12472 (Datenbankcode NP_901695) entspricht, eine andere Aminosäure als Threonin und bevorzugt eine Aminosäure aus der Gruppe umfassend Serin, Cystein, Glycin und Alanin aufweist.

In einer sechsten Ausführungsform des ersten Aspektes, die auch eine Ausführungsform der ersten bis fünften Ausführungsform darstellt, wird Schritt b) und/oder Schritt c) in Anwesenheit einer isolierten oder rekombinanten Alanindehydrogenase und einer anorganischen Stickstoffquelle durchgeführt.

In einer siebten Ausführungsform des ersten Aspektes, die auch eine Ausführungsform der ersten bis siebten Ausführungsform darstellt, ist wenigstens ein Enzym aus der Gruppe umfassend NAD(P)⁺-abhängige Alkoholdehydrogenase, Transaminase, Monooxygenase und Alanindehydrogenase rekombinant und wird in Form eines Ganzzellkatalysators bereitgestellt, der das entsprechende Enzym aufweist.

In einer achten Ausführungsform des ersten Aspektes, die eine Ausführungsform der siebten Ausführungsform darstellt, werden sämtliche Enzyme in Form von einem oder mehr als einem Ganzzellkatalysator bereitgestellt, wobei bevorzugt ein Ganzzellkatalysator sämtliche erforderliche Enzyme aufweist.

In einer neunten Ausführungsform des ersten Aspektes, die auch eine Ausführungsform der ersten bis achten Ausführungsform darstellt, ist bei Schritt b), bevorzugt bei Schritt b) und c), ein organisches Cosolvens anwesend, das einen logP von mehr als -1,38, bevorzugt 0 bis 1,2 aufweist.

In einer zehnten Ausführungsform des ersten Aspektes, die eine Ausführungsform der neunten Ausführungsform darstellt, ist das Cosolvens aus der Gruppe ausgewählt, die ungesättigte Fettsäuren, bevorzugt Ölsäure, umfasst.

In einer elften Ausführungsform des vierten Aspektes, die eine bevorzugte Ausführungsform der neunten Ausführungsform darstellt, ist das Cosolvens um eine Verbindung der Formel R³- O - (CH₂)ₓ - O - R⁴ handelt, wobei R³ und R⁴ jeweils und unabhängig einander aus der Gruppe ausgewählt sind, die Methyl, Ethyl, Propyl und Butyl umfasst und x 1 bis 4 ist, wobei besonders bevorzugt R³ und R⁴ jeweils Methyl und x 2 ist.

In einer zwölften Ausführungsform des ersten Aspektes, die eine bevorzugte Ausführungsform der neunten Ausführungsform darstellt, ist das Cosolvens aus der Gruppe ausgewählt, die Dialkylether umfasst und ist bevorzugt Dimethylether.
Erfindungsgemäß wird die Aufgabe in einem zweiten Aspekt durch die Verwendung des Ganzzellkatalysators aufweisend eine NAD(P)⁺-abhängige Alkoholdehydrogenase, bevorzugt mit wenigstens einem Zinkatom als Cofaktor, eine Transaminase, optional eine Monooxygenase und optional eine Alanindehydrogenase, wobei es sich bei den Enzymen um rekombinante Enzyme handelt zur Oxidation und Aminierung eines primären Alkohols der Formel HO - (CH₂)ₓ - R¹, wobei R¹ aus der Gruppe ausgewählt ist, die -OH, -SH, -NH₂ und -COOR² umfasst, x wenigstens 3 ist und R² aus der Gruppe ausgewählt ist, die H, Alkyl und Aryl umfasst, verwendet.

In einer ersten Ausführungsform des zweiten Aspekts, die eine Ausführungsform der ersten Ausführungsform darstellt, umfasst die Verwendung weiter die Anwesenheit eines organischen Cosolvens, das einen logP von mehr als -1,38, bevorzugt 0 bis 1,2 aufweist und bevorzugt Dimethylether ist.

In einer zweiten Ausführungsform des zweiten Aspekts, die eine Ausführungsform der zweiten Ausführungsform darstellt, ist das Cosolvens ausgewählt aus der Gruppe, die ungesättigte Fettsäuren umfasst, und ist bevorzugt Ölsäure.

Weitere Ausführungsformen des zweiten Aspektes umfassen sämtliche Ausführungsformen des ersten Aspektes der vorliegenden Erfindung.

Die Erfinder der vorliegenden Erfindung haben überraschend gefunden, dass es eine Gruppe von Alkoholdehydrogenasen gibt, die eingesetzt werden können, um die Oxidation von primären Alkoholen unter Entstehung geringerer Mengen von Nebenprodukten zu bewerkstelligen.

Die Erfinder haben weiterhin überraschend gefunden, dass eine Kaskade von enzymatischen Aktivitäten existiert, mit der Alkohole ohne nennenswerte Entstehung von Nebenprodukten unter Verwendung von Biokatalysatoren aminiert werden können, wobei keine Reduktionsäquivalente hinzugegeben oder abgeführt werden müssen.

Die Erfinder haben weiterhin überraschend ein Verfahren gefunden, mit dem Polyamide überraschend unter Verwendung eines Ganzzellkatalysators und ausgehend von nachwachsenden Rohstoffen hergestellt werden können.

Die Erfinder der vorliegenden Erfindung haben weiterhin überraschend gefunden, dass die Aminierung von primären Alkoholen nach vorheriger Oxidation besonders vorteilhaft mit einer durch bestimmte Sequenzeigenschaften gekennzeichneten Gruppe von Transaminasen durchgeführt werden kann.

Die Erfinder der vorliegenden Erfindung haben überraschend gefunden, dass in einem System zur Oxidation und Aminierung primärer Alkohole umfassend eine Alkoholdehydrogenase, eine Transaminase und eine Alanindehydrogenase die Verwendung einer anderen Alkoholdehydrogenase als einer vom Typ AlkJ, insbesondere die Verwendung einer NAD(P)+-abhängigen Alkoholdehydrogenasen mit Hinblick auf die Ausbeute des Verfahrens, besonders bei Durchführung unter Verwendung eines Ganzzellkatalysators, vorteilhaft ist.

Das erfindungsgemäße Verfahren kann auf eine große Anzahl von industriell relevanten Alkoholen angewandt werden. In einer bevorzugten Ausführungsform handelt es sich dabei um eine ω-Hydroxy-Carbonsäure oder einen Ester, bevorzugt Methylester, davon, die oder der zu einer ω-Aminocarbonsäure oxidiert und aminiert wird. In einer weiteren Ausführungsform handelt es sich um ein Diol, das zu einem Diamin oxidiert und aminiert wird. In einer weiteren bevorzugten Ausführungsform handelt es sich bei dem primären Alkohol um ein Hydroxyalkylamin. Die Länge der Kohlenstoffkette ist dabei variabel und x beträgt wenigstens 3. Bevorzugt weist die Kohlenstoffkette mehr als drei C-Atome auf, d. h. x = 4, 5, 6, 7, 8, 9, 10, 11, 12, 13, 14, 15, 16, 17, 18, 19, 20 oder mehr. Beispielhafte Verbindungen umfassen ω-Hydroxy-Laurinsäure, ω-Hydroxy-Laurinsäure-Methylester, und Alkan-Diole, insbesondere 1,8-Octan-Diol und 1,10-Dekan-Diol.

In einer besonders bevorzugten Ausführungsform ist R¹ aus der Gruppe ausgewählt, die -OH und -COOR² umfasst, x ist wenigstens 11 und R² ist aus der Gruppe ausgewählt, die H, Methyl, Ethyl und Propyl umfasst. In einer bevorzugtesten Ausführungsform ist handelt es sich bei dem primären Alkohol um einen ω-Hydroxy-Fettsäure-Methylester.

Erfindungsgemäß werden in Schritt b) des Verfahrens NAD(P)⁺-abhängige Alkoholdehydrogenasen zur Oxidation des primären Alkohols verwendet. Dabei kann es sich, wie bei allen erfindungsgemäß eingesetzten enzymatisch aktiven Polypeptiden, um Zellen umfassend enzymatisch aktive Polypeptide oder deren Lysate oder Präparationen der Polypeptide in sämtlichen Aufreinigungsstufen, vom kruden Lysat bis zum reinen Polypeptid, handeln. Dem Fachmann auf dem Gebiet sind zahlreiche Verfahren bekannt, mit denen enzymatisch aktive Polypeptide in geeigneten Zellen überexprimiert und aufgereinigt bzw. isoliert werden können. So können zur Expression der Polypeptide sämtliche dem Fachmann zur Verfügung stehende Expressionssysteme verwendet werden. Zur Aufreinigung kommen chromatographische Verfahren in Frage, beispielsweise die affinitätschromatographische Aufreinigung eines mit einem Tag versehenen rekombinanten Proteins unter Verwendungen eines immobilisierten Liganden, beispielsweise eines Nickelions im Falle eines Histidin-Tags, von immobilisiertem Glutathion im Falle einer an das Zielprotein fusionierten Glutathion-S-Transferase oder von immobilisierter Maltose im Falle eines Tags umfassend Maltosebindendes Protein.

Die aufgereinigten enzymatisch aktiven Polypeptide können entweder in löslicher Form oder immobilisiert eingesetzt werden. Dem Fachmann sind geeignete Verfahren bekannt, mit denen Polypeptide an organischen oder anorganischen Festphasen kovalent oder nichtkovalent immobilisiert werden können, beispielsweise durch Sulfhydryl-Kupplungs-Chemie (z.B. Kits der Firma Pierce).

In einer bevorzugten Ausführungsform handelt es sich bei der als Ganzzellkatalysator oder bei der als Expressionssystem verwendeten Zelle um eine prokaryotische, bevorzugt um eine bakterielle Zelle. In einer weiteren bevorzugten Ausführungsform handelt es sich um eine Säugetierzelle. In einer weiteren bevorzugten Ausführungsform handelt es sich um eine niedere eukaryotische Zelle, bevorzugt um eine Hefezelle. Beispielhafte prokaryotische Zellen umfassen *Escherichia,* besonders *Escherichia coli,* und Stämme der Gattung *Pseudomonas* und *Corynebacterium.* Beispielhafte niedere eukaryotische Zellen umfassen die Gattungen *Saccharomyces, Candida, Pichia, Yarrowia, Schizosaccharomyces,* besonders die Stämme *Candida tropicalis, Schizosaccharomyces pombe, Pichia pastoris, Yarrowia lipolytica* und *Saccharomyces cerivisiae.*

In einer besonders bevorzugten Ausführungsform handelt es sich bei der Alkoholdehydrogenase um eine zinkhaltige NAD(P)⁺-abhängige Alkoholdehydrogenase, d. h. das katalytisch aktive Enzym umfasst wenigstens ein Zink-Atom als Cofaktor, der durch ein charakteristisches Sequenzmotiv umfassend Cystein-Reste kovalent an das Polypeptid gebunden ist. In einer besonders bevorzugten Ausführungsform handelt es sich bei der Alkoholdehydrogenase um die Alkoholdehydrogenase von *Bacillus stearothermophilus* (Datenbankcode P42328) oder eine Variante davon.

Die Lehre der vorliegenden Erfindung kann nicht nur unter Verwendung der exakten Aminosäure- oder Nukleinsäuresequenzen der hierin beschriebenen biologischen Makromoleküle ausgeführt werden, sondern auch unter Verwendung von Varianten derartiger Makromoleküle, die durch Deletion, Addition oder Substitution einer oder mehr als einer Aminosäuren oder Nukleinsäuren erhalten werden können. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder letztere lediglich konservativ substituiert sind, beispielsweise ein Glutamat statt einem Aspartat oder ein Leucin statt einem Valin. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3rd edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatisch Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den K_{M}- und/oder k_{cat}- Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Alkoholdehydrogenase, Monooxygenase oder Transaminase. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in Ausubel *et al.* 1995 beschrieben. In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

Alkoholdehydrogenasen stellen eine seit Jahrzehnten in der Biochemie im Zusammenhang mit brauereitechnischen Fermentationsprozessen stark beachtete und biotechnologisch hochrelevante Enzymklasse dar, die verschiedene Gruppen von Isoformen umfasst. So existieren membrangebundene, flavinabhängige Alkoholdehydrogenasen vom *Pseudomonas putida* GPO1 AlkJ-Typ, die Flavocofaktoren statt NAD(P)⁺ verwenden. Eine weitere Gruppe umfasst eisenhaltige, gegenüber Sauerstoff empfindliche Alkoholdehydrogenasen, die in Bakterien und in inaktiver Form in Hefe gefunden werden. Eine andere Gruppe umfasst NAD(P)⁺-abhängige Alkoholdehydrogenasen, unter ihnen zinkhaltige Alkoholdehydrogenasen, bei denen das aktive Zentrum ein cysteinkoordiniertes Zinkatom aufweist, das das Alkoholsubstrat fixiert. In einer bevorzugten Ausführungsform wird unter dem Begriff "Alkoholdehydrogenase", wie hierin verwendet, wird ein Enzym verstanden, das einen Aldehyd bzw. Keton zu dem entsprechenden primären bzw. sekundären Alkohol oxidiert. Bevorzugt handelt es sich bei der Alkoholdehydrogenase im erfindungsgemäßen Verfahren um eine NAD(P)⁺-abhängige Alkoholdehydrogenase, d.h. eine Alkoholdehydrogenase, die NAD(P)⁺ als Cofaktor zur Oxidation des Alkohols bzw. NAD(P)H zur Reduktion des entsprechenden Aldehyds bzw. Ketons verwendet. In der bevorzugtesten Ausführungsform handelt es sich bei der Alkoholdehydrogenase um eine NAD(P)⁺-abhängige, zinkhaltige Alkoholdehydrogenase. In einer bevorzugten Ausführungsform bezeichnet der Begriff "NAD(P)⁺-abhängige Alkoholdehydrogenase", wie hierin verwendet, eine Alkoholdehydrogenase, die NAD⁺- und/oder NADP⁺-abhängig ist.

Erfindungsgemäß wird in Schritt c) eine Transaminase verwendet. In einer bevorzugten Ausführungsform wird unter dem Begriff "Transaminase", wie hierin verwendet, ein Enzym verstanden, das die Übertragung von α-Aminogruppen von einem Donor-, bevorzugt eine Aminosäure, auf ein Akzeptormolekül, bevorzugt eine α-Ketocarbonsäure, katalysiert. In einer bevorzugten Ausführungsform ist die Transaminase aus der Gruppe von Transaminasen und deren Varianten ausgewählt, die dadurch gekennzeichnet sind, dass sie an der Position der Aminosäuresequenz, die Val224 aus der Transaminase von *Chromobacterium violaceum* ATCC 12472 (Datenbankcode NP_901695) entspricht, eine Aminosäure ausgewählt aus der Gruppe umfassend Isoleucin, Valin, Phenylalanin, Methionin und Leucin aufweist, und an der Position der Aminosäuresequenz, die Gly230 aus der Transaminase von *Chromobacterium violaceum* ATCC 12472 (Datenbankcode NP_901695) entspricht, eine andere Aminosäure als Threonin und bevorzugt eine Aminosäure aus der Gruppe umfassend Serin, Cystein, Glycin und Alanin aufweist. In einer besonders bevorzugten Ausführungsform ist die Transaminase aus der Gruppe ausgewählt, die die ω-Transaminase aus *Chromobacterium violaceum* DSM30191, Transaminasen aus *Pseudomonas putida* W619, aus *Pseudomonas aeruginosa* PA01, *Streptomyces coelicolor* A3(2) und *Streptomyces avermitilis* MA 4680 umfasst.

In einer bevorzugten Ausführungsform bedeutet der Begriff "Position, die der Position X der Aminosäuresequenz aus der Transaminase von *Chromobacterium violaceum* ATCC 12472" entspricht, wie hierin verwendet, dass die entsprechende Position bei einem Alignment des untersuchten Moleküls als zur Position X der Aminosäuresequenz aus der Transaminase von *Chromobacterium violaceum* ATCC 12472 homolog erscheint. Dem Fachmann sind zahlreiche Softwarepakete und Algorithmen bekannt, mit denen ein Alignment von Aminosäuresequenzen angefertigt werden kann. Beispielhafte Softwarepakete Verfahren umfassen das vom EMBL bereitgestellte Paket ClustalW (Larkin *et al.,* 2007; Goujon *et al.* 2010) oder sind in Arthur M. Lesk (2008), Introduction to Bioinformatics, 3rd edition, aufgeführt und beschrieben.

Bei den erfindungsgemäß verwendeten Enzymen handelt es sich bevorzugt um rekombinante Enzyme. In einer bevorzugten Ausführungsform wird unter dem Begriff "rekombinant", wie hierin verwendet, verstanden, dass das entsprechende Nukleinsäure-Molekül in der Natur nicht vorkommt und/oder es unter Verwendung von gentechnischen Methoden hergestellt wurde. In einer bevorzugten Ausführungsform spricht man von einem rekombinanten Protein, wenn das entsprechende Polypeptid von einer rekombinanten Nukleinsäure kodiert ist. In einer bevorzugten Ausführungsform wird unter einer rekombinanten Zelle, wie hierin verwendet, eine Zelle verstanden, die wenigstens eine rekombinante Nukleinsäure oder ein rekombinantes Polypeptid aufweist. Dem Fachmann sind zum Herstellen rekombinanter Moleküle oder Zellen geeignete Verfahren bekannt, beispielsweise die in Sambrook *et al.,* 1989, beschriebenen.

Die erfindungsgemäße Lehre kann sowohl unter Verwendung von isolierten Enzymen als auch unter Verwendung von Ganzzellkatalysatoren ausgeführt werden. In einer bevorzugten Ausführungsform versteht man unter dem Begriff "Ganzzellkatalysator", wie hierin verwendet, eine intakte, lebensfähige und metabolisch aktive Zelle, die eine erwünschte enzymatische Aktivität bereitstellt. Der Ganzzellkatalysator kann das zu verstoffwechselnde Substrat, im Falle der vorliegenden Erfindung den Alkohol oder das daraus entstehende Oxidationsprodukt, entweder ins Zellinnere transportieren, wo es von cytosolischen Enzymen verstoffwechselt wird, oder er kann das Enzym von Interesse auf seiner Oberfläche präsentieren, wo es gegenüber Substraten im Medium direkt exponiert ist. Dem Fachmann sind zahlreiche Systeme zur Herstellung von Ganzzellkatalysatoren bekannt, beispielsweise aus der DE 60216245.

Für eine Reihe von Anwendungen empfiehlt sich die Verwendung isolierter Enzyme. In einer bevorzugten Ausführungsform bedeutet der Begriff "isoliert", wie hierin verwendet, dass das Enzym in reinerer und/oder aufkonzentrierter Form vorliegt als in seiner natürlichen Quelle. In einer bevorzugten Ausführungsform gilt das Enzym als isoliert, wenn es ein Polypeptidenzym ist und mehr als 60, 70, 80, 90 oder bevorzugt 95 % des Massenproteinanteils der entsprechenden Präparation ausmacht. Dem Fachmann sind zahlreiche Verfahren zur Messung der Masse eines Proteins in einer Lösung bekannt, beispielsweise die visuelle Abschätzung anhand der Dicke von entsprechenden Proteinbanden auf SDS-Polyacrylamidgelen, NMR-Spektroskopie oder massenspektrometriebasierte Verfahren.

Die enzymatisch katalysierten Reaktionen des erfindungsgemäßen Verfahrens werden typischerweise in einem Lösungsmittel oder Lösungsmittelgemisch mit hohem Wasseranteil, bevorzugt in Anwesenheit eines geeigneten Puffersystems zur Einstellung eines mit enzymatischer Aktivität kompatiblen pH-Wertes, ausgeführt. Im Falle hydrophober Edukte, insbesondere bei Alkoholen mit einer Kohlenstoffkette umfassend mehr als drei Kohlenstoffatome, ist jedoch die zusätzliche Anwesenheit eines organischen Cosolvens vorteilhaft, welches den Kontakt des Enzyms mit dem Substrat vermitteln kann. Das einer oder mehr als eine Cosolvens liegt in einem Gesamtanteil am Lösungsmittelgemisch von oder weniger als 95, 90, 85, 80, 75, 70, 65, 60, 55, 50 45, 40, 35, 30, 25, 20, 15, 10 oder 5 Volumenprozent vor.

Die Hydrophobizität des Cosolvens spielt dabei eine gewichtige Rolle. Sie lässt sich durch den logP, den dekadischen Logarithmus des n-Oktanol-Wasser-Verteilungskoeffizienten darstellen. Ein bevorzugtes Cosolvens weist einen logP von mehr als -1,38 auf, bevorzugter von -1 bis +2, noch bevorzugter von 0 bis 1,5.

Der n-Oktanol-Wasser-Verteilungskoeffizient K^{ow} oder P ist ein dimensionsloser Verteilungskoeffizient, der das Verhältnis der Konzentrationen eines Stoffes in einem Zweiphasensystem aus 1-Oktanol und Wasser angibt (siehe J. Sangster, Octanol-Water Partition Coefficients: Fundamentals and Physical Chemistry, Vol. 2 of Wiley Series in Solution Chemistry, John Wiley & Sons, Chichester, 1997). Genauer gesagt bezeichnet der K^{ow} oder P das Verhältnis der Konzentration des Stoffes in der oktanolreichen Phase zu seiner Konzentration in der wasserreichen Phase.

Der K^{ow}-Wert ist ein Modellmaß für das Verhältnis zwischen Lipophilie (Fettlöslichkeit) und Hydrophilie (Wasserlöslichkeit) einer Substanz. Es besteht die Erwartung, mit Hilfe des Verteilungskoeffizienten eines Stoffes im System Oktanol-Wasser auch die Verteilungskoeffizienten dieses Stoffes in anderen Systemen mit einer wässrigen Phase abschätzen zu können. K^{ow} ist größer als eins, wenn eine Substanz besser in fettähnlichen Lösungsmitteln wie n-Oktanol löslich ist, kleiner als eins wenn sie besser in Wasser löslich ist. Entsprechend ist Log P positiv für lipophile und negativ für hydrophile Substanzen. Da nicht für alle Chemikalien der K^{OW} gemessen werden kann, gibt es verschiedenste Modelle für die Vorhersage, z. B. durch Quantitative Struktur-Aktivitäts-Beziehungen (QSAR) oder durch Linear Free Energy Relationships (LFER), beispielsweise beschrieben in Eugene Kellogg G, Abraham DJ: Hydrophobicity: is LogP(o/w) more than the sum of its parts?. Eur J Med Chem. 2000 Jul-Aug;35(7-8):651-61 oder Gudrun Wienke, "Messung und Vorausberechnung von n-Octanol/Wasser-Verteilungskoeffizienten", Doktorarbeit, Univ. Oldenburg, 1-172, 1993.

Im Rahmen der vorliegenden Erfindung wird log P nach der Methode von Advanced Chemistry Development Inc., Toronto mittels des Programmmoduls ACD/LogP DB bestimmt.

Ein bevorzugtes Cosolvens weist einen logP von mehr als -1,38 auf, bevorzugter von -1 bis +2, noch bevorzugter von -0,75 bis 1,5 oder -0,5 bis 0,5 oder -0,4 bis 0,4 oder -0,3 bis -0,1. In einer bevorzugten Ausführungsform handelt es sich bei dem Cosolvens um einen Dialkylether der Formel Alk₁-O-Alk₂ mit einem einen logP von mehr als -1,38 auf, bevorzugter von -1 bis +2, noch bevorzugter von 0 bis 1,5, wobei die beiden Alkylsubstituenten Alk₁ und Alk₂ jeweils und unabhängig voneinander aus der Gruppe ausgewählt sind, die Methyl, Ethyl, Propyl, Butyl, Isopropyl und tert-Butyl umfasst. In einer besonders bevorzugten Ausführungsform handelt es sich bei dem Cosolvens um Methyltertiärbutylether (MTBE). In der bevorzugtesten Ausführungsform handelt es sich bei dem Cosolvens um Dimethoxyethan (DME). In einer weiteren bevorzugten Ausführungsform es sich bei dem Cosolvens um eine Verbindung der Formel R³ - O - (CH₂)ₓ - O - R⁴ handelt, wobei R³ und R⁴ jeweils und unabhängig einander aus der Gruppe ausgewählt sind, die Methyl, Ethyl, Propyl und Butyl umfasst und x 1 bis 4 ist, wobei bevorzugt R³ und R⁴ jeweils Methyl und x 2 ist.

In einer weiteren bevorzugten Ausführungsform handelt es sich bei dem Cosolvens um eine Carbonsäure oder Fettsäure, bevorzugt eine Fettsäure mit wenigstens 6, bevorzugter wenigstens 12 Kohlenstoffatomen. Die Fettsäure kann eine gesättigte Fettsäure sein, beispielsweise Laurinsäure, Myristinsäure, Palmitinsäure, Margarinsäure, Stearinsäure, Arachinsäure oder Behensäure, oder eine ungesättigte, beispielsweise Myristoleinsäure, Palmitoleinsäure, Petroselinsäure, Ölsäure, Elaidinsäure, Vaccensäure, Gadoleinsäure, Icosensäure oder Erucasäure. Ebenso möglich sind Mischungen verschiedener Fettsäuren, beispielweise Kugeldistelöl, das hauptsächlich ungesättigte Fettsäuren enthält. Da nicht alle Fettsäuren in nennenswertem Maß bei Raumtemperatur löslich sind, kann es notwendig sein, weitere Maßnahmen zu ergreifen, wie beispielsweise die Erhöhung der Temperatur oder, bevorzugterweise, die Zugabe eines weiteren Lösungsmittels, um sie der wässrigen Phase zugänglich zu machen. In einer besonders bevorzugten Ausführungsform findet eine Fettsäure oder ein Ester davon, bevorzugt der Methylester, am bevorzugtesten Laurinsäuremethylester, als ein solches weiteres Lösungsmittel Verwendung.

Die erfindungsgemäße enzymatische Kaskade kann erfindungsgemäß in Anwesenheit einer Alanindehydrogenase ablaufen. Es ist eine besondere Stärke der vorliegenden Erfindung, dass diese Ausgestaltung eine reduktionsäquivalentneutrale Reaktionsführung ermöglicht, d.h. die Reaktion läuft ohne Zufuhr oder Entfernung von Elektronen in Form von Reduktionsäquivalenten ab, da die von der Alkoholdehydrogenase im Zuge der Alkoholoxidation erzeugte NADH bei der Erzeugung von Alanin unter Verbrauch eines anorganischen Stickstoffdonors, bevorzugt Ammoniak oder eine Ammoniakquelle, verbraucht wird.

In einer bevorzugten Ausführungsform wird unter dem Begriff "Alanindehydrogenase", wie hierein verwendet, ein Enzym verstanden, das die Umwandlung von L-Alanin unter Verbrauch von Wasser und NAD⁺ zu Pyruvat, Ammoniak und NADH katalysiert. Bevorzugt handelt es sich bei der Alanindehydrogenase um eine intrazelluläre Alanindehydrogenase, noch bevorzugter um eine rekombinante intrazelluläre Alanindehydrogenase eines bakteriellen Ganzzellkatalysators.

In einer bevorzugten Ausführungsform wird für das erfindungsgemäße Verfahren ein Ganzzellkatalysator mit allen erforderlichen Aktivitäten eingesetzt, d.h. NAD(P)⁺-abhängige Alkoholdehydrogenase, Transaminase und ggf. Monooxygenase und/oder Alanindehydrogenase. Die Verwendung eines derartigen Ganzzellkatalysators hat den Vorteil, dass sämtliche Aktivitäten in Form von einem einzelnen Agens eingesetzt werden und es nicht notwendig ist, Enzyme in biologisch aktiver Form großtechnisch aufzubereiten. Dem Fachmann sind geeignete Verfahren zur Konstruktion von Ganzzellkatalysatoren bekannt, insbesondere die Konstruktion von Plasmidsystemen zur Expression von einem oder mehr als einem rekombinanten Protein oder die Integration der für die erforderlichen rekombinanten Protein kodierenden DNA in die chromosomale DNA der verwendenden Wirtszelle.

Die in der vorangehenden Beschreibung, den Ansprüchen und den Zeichnungen offenbarten Merkmale der Erfindung können sowohl einzeln als auch in beliebiger Kombination zur Verwirklichung der Erfindung in ihren verschiedenen Ausführungsformen wesentlich sein.

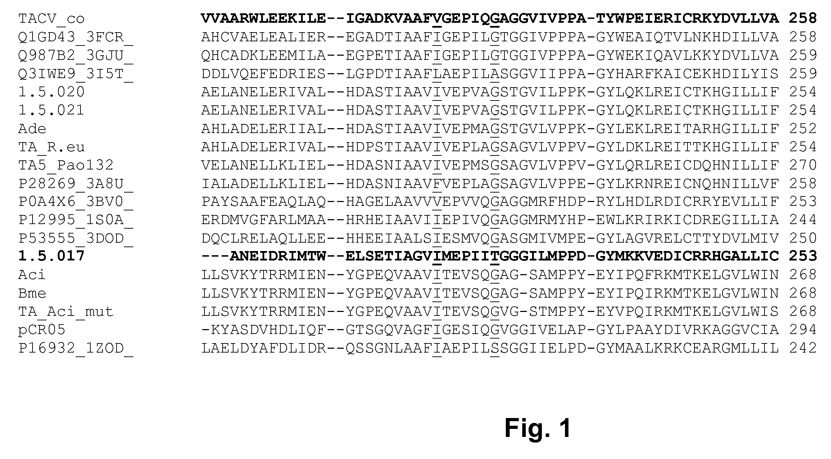

**Fig. 1** zeigt ein beispielhaftes Alignment umfassend verschiedene Transaminasen, insbesondere die von *Chromobacterium violaceum* ATCC 12472 (Datenbankcode NP_901695, "TACV_co"). Die den Positionen Val224 und Gly230 letzterer Transaminase entsprechenden Aminosäurereste sind in allen Sequenzen unterstrichen. Das Alignment wurde unter Verwendung von Clustal W2 angefertigt.

### Beispiel 1: Aminierung verschiedener Substrate unter Verwendung einer NAD⁺-abhängigen Alkoholdehydrogenase im Vergleich zur Alkoholdehydrogenase AlkJ unter Verwendung des erfindungsgemäßen Verfahrens

### Substrates:

Als Substrate wurden Hexan-1,6-Diol **(1),** 6-Aminohexan-1-ol **(2)** und Ethyl-6-Hydroxyhexanoat **(3)** verwendet.

### Enzyme:

### Alanin-Dehydrogenase:

Die L-Alanin-Dehydrogenase von *Bacillus subtilis* wurde in *E. coli* exprimiert. Zunächst wurde eine Übernachtkultur vorbereitet, die anschließend verwendet wurde, um die Hauptkultur (LB-Ampicillin-Medium) anzuimpfen. Die Zellen wurden 24 Stunden lang bei 30 °C und 120 Upm auf einem Schüttler inkubiert. Anschließend wurde unter sterilen Bedingungen IPTG (0,5 mM, Isopropyl β-D-1-thiogalactopyranoside, Sigma) zur Induktion hinzugegeben, und die Kulturen wurden weitere 24 Stunden lang bei 20 °C geschüttelt.

Die Zellen wurden abzentrifugiert (8000 Upm, 20 min 4 °C), gewaschen und der Überstand verworfen. Anschließend wurden die Zellen unter Verwendung von Ultraschall (1 s Puls, 4 s Pause, Zeit: 10 min, Amplitude: 40 %) disruptiert, die Mischung wurde abzentrifugiert (20 min, 18000 Upm, 4 °C) und das Enzym unter Verwendung einer His-prep-Säule aufgereinigt.

### Alkoholdehydrogenase von Bacillus stearothermophilus (ADH-hT; P42328.1))

Zur Präparation der NAD⁺-abhängigen Alkoholdehydrogenase von *Bacillus stearothermophilus* (Fiorentino G, Cannio R, Rossi M, Bartolucci S: Decreasing the stability and changing the substrate specificity of the Bacillus stearothermophilus alcohol dehydrogenase by single amino acid replacements. Protein Eng 1998, 11: 925-930) wurde zunächst eine Übernachtkultur vorbereitet (10 ml LB/Ampicillin-Medium, ampicilline 100µg/ml, 30°C, 120 Upm), die anschließend verwendet wurde, um Kulturgefäße anzuimpfen, die wiederum etwa 12 Stunden lagen bei 37 °C und 120 Upm geschüttelt wurden. Die Zellen wurden abzentrifugiert (8000 Upm, 20 Minuten, 4 °C), gewaschen, der Überstand verworfen und das Pellet lyophilisiert. Schließlich wurden die Zellen unter Verwendung von Ultraschall (1 s Puls, 4 s Pause, Zeit: 10 min, Amplitude: 40 %) disruptiert, die Mischung wurde abzentrifugiert (20 min, 18000 Upm, 4 °C) und als krudes Extrakt verwendet. Die Proteinkonzentration wurde mittels SDS-PAGE abgeschätzt.

### AlkJ-Alkoholdehydrogenase (aus Pseudomonas oleovirans Gpo1):

Das Enzym wurde unter den gleichen Bedingungen präpariert wie die Alkoholdehydrogenase von *Bacillus stearothermophilus,* abgesehen davon, dass das Plasmid pTZE03_AlkJ (SEQ ID NO 20) und Kanamycin als Antibiotikum (50 µg/ml) verwendet wurde. Die Proteinkonzentration wurde ebenfalls mittels SDS-PAGE abgeschätzt.

### Transaminase CV-ωTA aus Chromobacterium violaceum:

Zur Präparation der CV-ωTA aus *Chromobacterium violaceum* (U. Kaulmann, K. Smithies, M. E. B. Smith, H. C. Hailes, J. M. Ward, Enzyme Microb. Technol. 2007, 41, 628-637; b) M. S. Humble, K. E. Cassimjee, M. Häkansson, Y. R. Kimbung, B. Walse, V. Abedi, H.-J. Federsei, P. Berglund, D. T. Logan, FEBS Journal 2012, 279, 779-792; c) D. Koszelewski, M. Göritzer, D. Clay, B. Seisser, W. Kroutil, ChemCatChem 2010, 2, 73-77) wurde Zunächst eine Übernachtkultur vorbereitet (LB/Ampicillin-Medium, 30°C, 120 rpm), die anschließend verwendet wurde, um Kulturflaschen mit dem gleichen Medium anzuimpfen, die etwa drei Stunden bei 37 °C und 120 Upm geschüttelt wurden, bis eine optische Dichte bei 600 nm von 0,7 erreicht wurde. Anschließend wurde IPTG-Stammlösung (0,5 mM) hinzugegeben und drei Stunden lang bei 20 °C und 120 Upm induziert. Die Zellen wurden abzentrifugiert, der Überstand verworfen und die Zellen bei 4 °C gelagert. Schließlich wurden die Zellen unter Verwendung von Ultraschall (1 s Puls, 4 s Pause, Zeit: 10 min, Amplitude: 40 %) disruptiert, die Mischung wurde abzentrifugiert (20 min, 18000 Upm, 4 °C) und der Überstand als krudes Extrakt verwendet.

### Versuchsdurchführung:

Der Versuchsansatz ist in Tab. 1 beschrieben.

**Tabelle 1: Versuchsansatz**

| | | |
|---|---|---|
| **Versuchsansatz** | ADH-hT (krude) | 200 µl |
| | Transaminase | 200 µl |
| | AlaDH | 10 µl (250 U) |
| | L-Alanin | 22.3 mg (250 µmol) |
| | NAD⁺ | 0.5 mg (0.75 µmol) |
| | NH₄Cl | 21 mg (500 µmol) |
| | PLP | 0.1 mg (0.35 µmol) |
| | NaOH 6 M | 7.5 µl |
| | H₂O / Cosolvens | 400 µl |
| | Substrat | 50 µmol |
| | | |
| | pH am Ende | 8.5 |
| | Gesamtvolumen | 1.22 mL |

Das Substrat wird in der entsprechenden Menge Kosolvenz (siehe **Tab. 2**) gelöst, und in 300 µl Wasser gelöstet L-Alanin wurde zugefügt. In 75 µl Wasser Ammoniumchlorid wurde hinzugegeben. Jeweils in 25 µl Wasser aufgelöstes NAD+ und PLP wurde hinzugegeben. Der pH-Wert wurde durch zugaben von 7,5 µl einer 6 M NaOH-Lösung eingestellt. Die Transaminase und Alanindehydrogenase wurden zugegeben. Die Reaktion wurde durch Zugabe von Alkohldehydrogenase gestartet. Nach 22 Stunden wurde die Reaktion durch Zugabe der unten angegebenen Derivatisierungs-Reagenzien gestoppt.

### Derivatisierung von Aminen:

Zu einer Probe mit 500 µl wurden 200 µl Triethylamin sowie ESOF (Ethyl-Succinimidooxyformiat) (80 oder 40 mg) in Acetonitril (500 µl) gegeben. Die Proben wurden anschließend eine Stunde lang bei 45 °C geschüttelt und anschließend mit Dichlormethan extrahiert, über Natriumsulfat getrocknet und unter Verwendung von GC-MS gemessen. Wurde ohne Alanindehydrogenase gearbeitet, so wurden zu einer wässrigen Lösung L-Alanine (500 mM), NAD⁺ (2 mM) und PLP (0.5 mM) bei einem pH-Wert von 8.5 (eingestellt durch Zugabe von NaOH) und Substrat in DME (120 µl, 25 mM) gegeben. Die Reaktion wurde durch Zugabe von jeweils 200 µl Alkoholdehydrogenase (NAD⁺-abhängig) oder AlkJ) und Transaminase gestartet. Die Proben wurden bei 25 °C und 300 Upm 24 Stunden lang geschüttelt. Die Proben wurden wie oben beschrieben aufgearbeitet und mit GC-MS analysiert.

### Ergebnisse:

**Tabelle 2. Aminierung in Abwesenheit von Alanindehydrogenase. Die Substrate sind Hexan-1,6-Diol (1), 6-Aminohexan-1-ol (2) und Ethyl-6-Hydroxyhexanoat (3).**

| Substrat | Oxidierundes Enzym | Transaminase | Nicht umgesetztes Substrat [%] | Produkt [%] |
|---|---|---|---|---|
| **1** | AlkJ | CV | >99 | <1 (Monoamin) |
| | | | | <1 (Diamin) |
| **1** | ADH-hT | CV | <1 | <99 (Monoamin) |
| | | | | >1 (Diamin) |
| **1** | - | - | >99 | <1 |
| **2** | AlkJ | CV | 91 | 9 |
| **2** | ADH-hT | CV | 76 | 24 |
| **2** | - | - | >99 | <1 |
| **3** | AlkJ | CV | 99 | 1 |
| **3** | ADH-hT | CV | 22 | 78 |
| **3** | - | - | >99 | <1 |

**Tabelle 3. Aminierung in Anwesenheit von Alanindehydrogenase. Die Substrate sind Hexan-1,6-Diol (1), 6-Aminohexan-1-ol (2) und Ethyl-6-Hydroxyhexanoat (3).**

| Substrat | Oxidierendes Enzym | Transaminase | Nicht umgesetztes Substrat [%] | Produkt [%] |
|---|---|---|---|---|
| **1** | AlkJ | CV | >99 | <1 (Monoamin) |
| | | | | <1 (Diamin) |
| **1** | ADH-hT | CV | <1 | 92 (Monoamin) |
| | | | | 8 (Diamin) |
| **1** | - | - | >99 | <1 (Monoamin) |
| | | | | <1 (Diamin) |
| **2** | AlkJ | CV | >99 | <1 |
| **2** | ADH-hT | CV | 19 | 81 |
| **2** | - | - | >99 | <1 |
| **3** | AlkJ | CV | >99 | <1 |
| **3** | ADH-hT | CV | <1 | >99 |
| **3** | - | - | >99 | <1 |

### Zusammenfassung:

Für eine Reihe von strukturell verschiedenen Substraten, nämlich Hexan-1,6-Diol **(1),** 6-Aminohexan-1-ol **(2)** und Ethyl-6-Hydroxyhexanoat **(3),** wurde jeweils gezeigt, dass die Reaktion unter Verwendung der NAD⁺-abhängigen Alkoholdehydrogenase von *Bacillus stearothermophilus* deutlich effizienter abläuft als unter Verwendung der Alkoholhydrogenase AlkJ. Dieser überraschende technische Effekt konnte gleichermaßen in Anwesenheit und Abwesenheit der Alanindehydrogenase gezeigt werden.

### Beispiel 2: Aminierung verschiedener Alkoholsubstrate

Um zu bestätigen, dass das erfindungsgemäße Enzymsystem strukturell vielfältige Substrate umsetzt, wurden weitere Alkanole und Alkandiole unter Verwendung von geeigneten Enzymen *in vitro* zu den entsprechenden Aminen bzw. Diaminen umgesetzt. Es stellte sich heraus, dass die Alkoholdehydrogenase aus Pferdeleber (HL-ADH, E-isoenzyme; NP_001075997.1) und die Alkoholdehydrogenase aus *Bacillus stearothermophilus* (ADH-hT; P42328.1) gleichermaßen geeignet sind. Zwei verschiedene Aminasen, nämlich die aus *Chromobacterium violaceum*^{[16]} (CV-ωTA) und eine Variante einer (S)-selektiven ω-TA aus *Arthrobacter citreus* (ArS-ωTA) (A. R. Martin, R. DiSanto, I. Plotnikov, S. Kamat, D. Shonnard, S. Pannuri, Biochem. Eng. J. 2007, 37, 246-255) wurden verwendet. Die Alanindehydrogenase stammte aus *Bacillus subtilis* (F. G. Mutti, C. S. Fuchs, D. Pressnitz, J. H. Sattler, W. Kroutil, Adv. Synth. Catal. 2011, 353, 3227-3233).

Die Produkte wurden mit Ethyl(succinimidooxy)format (I. Edafiogho, K. R. Scott, J. A. Moore, V. A. Famar, J. M. Nicholson, J. Med. Chem. 1991, 34, 387-392) derivatisiert und durch GC-MS unter Verwendung einer Agilent J&W HP-5-Säule (30 m, 320 µm, 0.25 µm) nachgewiesen.

**Tabelle 4.: Aminierung von primären Alkoholen**

| Nr. | Substrat | c [%] | Aldehyd [%] | Amine [%] |
|---|---|---|---|---|
| 1 | 1-Hexanol | >99 | <1 | > 99 |
| 2 | 1-Octanol | 50 | <1 | 50 |
| 3 | 1-Octanol | 57^{[b]} | <1 | 57 |
| 4 | 1-Decanol | 2 | <1 | 2 |
| 5 | 1-Decanol | 25 ^{[b]} | <1 | 25 |
| 6 | 1-Dodecanol | 10^{[b]} | <1 | 10 |

| | | | | |
|---|---|---|---|---|
| [a] Reaktionsbedingungen: Substrat (50 mM), CV-ωTA (1 mg, 0.2 U) und ADH-hT (1 mg, 0.25 U), AlaDH (0.0.4 mg, 0.25 U), PLP (0.35 mM), NAD⁺ (0.75 mM), Ammoniumchlorid (275 mM), L-Alanine (250 mM), pH 8,5, 24 hours, 20 °C. [b] 1,2-Dimethoxyethan (10% v v⁻¹) wurde als Cosolvens zugegeben. | | | | |

**Tabelle 5. Aminierung von 1,ω-Diolen^{[c]}**

| No. | Sub. | Solvent | Vol. Solv. [%] | T. [°C] | c [%] | **Monoamin** [%] | **Diamin** [%] |
|---|---|---|---|---|---|---|---|
| 1 | **1,8-Octandiol** | MTBE | 30 | 35^{[d]} | 52 | 49 | 3 |
| 2 | **1,8-Octandiol** | MTBE | 30 | 25 | 98 | 52 | 46 |
| 3 | **1,8-Octandiol** | DME | 40 | 25 | 95 | 80 | 15 |
| 4 | **1,8-Octandiol** | DME | 30 | 25 | >99 | 18 | 82 |
| 5 | **1,8-Octandiol** | DME | 20 | 25 | 99 | 16 | 83 |
| 6 | **1,8-Octandiol** | DME | 10 | 25 | 99 | 1 | 98 |
| 7 | **1,8-Octandiol** | DME | 10 | 20 | >99 | <1 | >99 |
| 8 | **1,10-DekanDiol** | DME | 20 | 25 | 99 | 6 | 93 |
| 9 | **1,10-DekanDiol** | DME | 20 | 20 | >99 | 2 | 98 |
| 10 | **1,10-DekanDiol** | DME | 10 | 20 | >99 | 1 | 99 |

| | | | | | | | |
|---|---|---|---|---|---|---|---|
| [c] Allgemeine Reaktionsbedingungen: Substrat (50 mM), CV-ωTA (1 mg, 0.25 U) and ADH-hT (1 mg, 0.2 U), AlaDH (0.1 mg, 0.7 U), PLP (0.35 mM), NAD⁺ (0.75 mM), Ammoniumchlorid (275 mM), L-Alanin (250 mM), pH 8.5. [d] ArS-ωTA verwendet statt CV-ωTA. | | | | | | | |

### Literaturstellen:

PCT/EP/2008/067447 (2009): ω-AMINO CARBOXYLIC ACIDS, ω-AMINO CARBOXYLIC ACID ESTERS, OR RECOMBINANT CELLS WHICH PRODUCE LACTAMS THEREOF
C. Grant, J. M. Woodley and F. Baganz (2011), Enzyme and Microbial Technology 48, 480-486
Gudrun Wienke, "Messung und Vorausberechnung von n-Octanol/Wasser-Verteilungskoeffizienten", Doktorarbeit, Univ. Oldenburg, 1-172, 1993
DE 60216245 (2007): FUNKTIONELLES OBERFLÄCHENDISPLAY VON POLYPEPTIDEN
Sambrook/Fritsch/Maniatis (1989): Molecular Cloning: A Laboratory Manual, Cold Spring Harbor Laboratory Press, 2nd edition
J. Sangster, Octanol-Water Partition Coefficients: Fundamentals and Physical Chemistry, Vol. 2 of Wiley Series in Solution Chemistry, John Wiley & Sons, Chichester, 1997
Eugene Kellogg G, Abraham DJ: Hydrophobicity: is LogP(o/w) more than the sum of its parts?. Eur J Med Chem. 2000 Jul-Aug;35(7-8):651-61
Larkin MA, Blackshields G, Brown NP, Chenna R, McGettigan PA, McWilliam H, Valentin F, Wallace IM, Wilm A, Lopez R, Thompson JD, Gibson TJ and Higgins DG Bioinformatics 2007 23(21): 2947-2948.
Goujon M, McWilliam H, Li W, Valentin F, Squizzato S, Paern J, Lopez R (2010) Nucleic acids research Jul, 38 Suppl: W695-9

## Patentansprüche

1. Verfahren zur Oxidation von Alkoholen umfassend die Schritte
a) Bereitstellen eines primären Alkohols der Formel
HO-(CH₂)ₓ-R¹,
wobei R¹ aus der Gruppe ausgewählt ist, die -OH, -SH, -NH₂ und -COOR² umfasst, x wenigstens 3 ist und R² aus der Gruppe ausgewählt ist, die H, Alkyl und Aryl umfasst,
b) Oxidation des primären Alkohols durch Kontaktieren mit einer NAD(P)⁺-abhängigen Alkoholdehydrogenase, und
c) Kontaktieren des Oxidationsproduktes aus Schritt a) mit einer Transaminase,
wobei es sich bei der NAD(P)⁺-abhängigen Alkoholdehydrogenase und/oder der Transaminase um ein rekombinantes oder isoliertes Enzym handelt.

2. Verfahren nach Anspruch 1, wobei Schritt a) durch Hydroxylierung eines Alkans der Formel
H-(CH₂)ₓ-R¹
durch eine Monooxygenase erfolgt, die bevorzugt rekombinant oder isoliert ist.

3. Verfahren nach einem der Ansprüche 1 bis 2, wobei es sich bei der NAD(P)⁺-abhängigen Alkoholdehydrogenase um eine NAD(P)⁺-abhängige Alkoholdehydrogenase mit wenigstens einem Zinkatom als Cofaktor handelt.

4. Verfahren nach Anspruch 3, wobei es sich bei der Alkoholdehydrogenase um die Alkoholdehydrogenase von *Bacillus stearothermophilus* (Datenbankcode P42328) oder eine Variante davon handelt.

5. Verfahren nach einem der Ansprüche 2 bis 4, wobei die Monooxygenase aus der Gruppe ausgewählt ist, die AlkBGT von *Pseudomonas putida,* Cytochrom P450 aus *Candida tropicalis* oder aus *Cicer arietinum* umfasst.

6. Verfahren nach einem der Ansprüche 1 bis 5, wobei die Transaminase aus der Gruppe von Transaminasen und deren Varianten ausgewählt ist, die **dadurch gekennzeichnet sind, dass** sie an der Position der Aminosäuresequenz, die Val224 aus der Transaminase von *Chromobacterium violaceum* ATCC 12472 (Datenbankcode NP_901695) entspricht, eine Aminosäure ausgewählt aus der Gruppe umfassend Isoleucin, Valin, Phenylalanin, Methionin und Leucin aufweist, und an der Position der Aminosäuresequenz, die Gly230 aus der Transaminase von *Chromobacterium violaceum* ATCC 12472 (Datenbankcode NP_901695) entspricht, eine andere Aminosäure als Threonin und bevorzugt eine Aminosäure aus der Gruppe umfassend Serin, Cystein, Glycin und Alanin aufweist.

7. Verfahren nach einem der Ansprüche 1 bis 6, wobei Schritt b) und/oder Schritt c) in Anwesenheit einer isolierten oder rekombinanten Alanindehydrogenase und einer anorganischen Stickstoffquelle durchgeführt wird.

8. Verfahren nach einem der Ansprüche 1 bis 7, wobei wenigstens ein Enzym aus der Gruppe umfassend NAD(P)⁺-abhängige Alkoholdehydrogenase, Transaminase, Monooxygenase und Alanindehydrogenase rekombinant ist und in Form eines Ganzzellkatalysators bereitgestellt wird, der das entsprechende Enzym aufweist.

9. Verfahren nach Anspruch 8, wobei sämtliche Enzyme in Form von einem oder mehr als einem Ganzzellkatalysator bereitgestellt werden und bevorzugt ein Ganzzellkatalysator sämtliche erforderliche Enzyme aufweist.

10. Verfahren nach einem der Ansprüche 1 bis 9, wobei bei Schritt b), bevorzugt bei Schritt b) und c), ein organisches Cosolvens anwesend ist, das einen logP von mehr als -1,38, bevorzugt 0 bis 1,2 aufweist

11. Verfahren nach Anspruch 10, wobei das Cosolvens aus der Gruppe ausgewählt ist, die ungesättigte Fettsäuren, bevorzugt Ölsäure, umfasst.

12. Verfahren nach Anspruch 10, wobei es sich bei dem Cosolvens um eine Verbindung der Formel R³-O-(CH₂)ₓ-O- R⁴ handelt, wobei R³ und R⁴ jeweils und unabhängig einander aus der Gruppe ausgewählt sind, die Methyl, Ethyl, Propyl und Butyl umfasst und x 1 bis 4 ist, wobei bevorzugt R³ und R⁴ jeweils Methyl und x 2 ist.

13. Verwendung eines Ganzzellkatalysators aufweisend eine NAD(P)⁺-abhängige Alkoholdehydrogenase, bevorzugt mit wenigstens einem Zinkatom als Cofaktor, eine Transaminase, optional eine Monooxygenase und optional eine Alanindehydrogenase, wobei es sich bei den Enzymen um rekombinante Enzyme handelt zur Oxidation und Aminierung eines primären Alkohols der Formel HO - (CH₂)ₓ - R¹, wobei R¹ aus der Gruppe ausgewählt ist, die -OH, -SH, -NH₂ und -COOR² umfasst, x wenigstens 3 ist und R² aus der Gruppe ausgewählt ist, die H, Alkyl und Aryl umfasst.

14. Verwendung nach Anspruch 13, weiter umfassend die Anwesenheit organischen Cosolvens anwesend ist, das einen logP von mehr als -1,38, bevorzugt 0 bis 1,2 aufweist.

15. Verwendung nach Anspruch 14, wobei das Cosolvens aus der Gruppe ausgewählt ist, die ungesättigte Fettsäuren, bevorzugt Ölsäure, umfasst.

## Claims

1. Method for oxidizing alcohols, comprising the steps
a) providing a primary alcohol of the formula
HO-(CH₂)ₓ-R¹,
wherein R¹ is selected from the group consisting of -OH, -SH, -NH₂ and -COOR², x is at least 3 and R² is selected from the group consisting of H, alkyl and aryl,
b) oxidizing the primary alcohol by contacting it with an NAD(P)⁺-dependent alcohol dehydrogenase, and
c) contacting the oxidation product of step a) with a transaminase,
wherein the NAD(P)⁺-dependent alcohol dehydrogenase and/or the transaminase is a recombinant or isolated enzyme.

2. Method according to Claim 1, wherein step a) proceeds by hydroxylating an alkane of the formula H-(CH₂)ₓ-R¹
by a monooxygenase which is preferably a recombinant or isolated monooxygenase.

3. Method according to either of Claims 1 and 2, wherein the NAD(P)⁺-dependent alcohol dehydrogenase is an NAD(P)⁺-dependent alcohol dehydrogenase having at least one zinc atom as cofactor.

4. Method according to Claim 3, wherein the alcohol dehydrogenase is an alcohol dehydrogenase from *Bacillus stearothermophilus* (database code P42328) or a variant thereof.

5. Method according to any one of Claims 2 to 4, wherein the monooxygenase is selected from the group consisting of AlkBGT from *Pseudomonas putida,* cytochrome P450 from *Candida tropicalis,* or from *Cicer arietinum.*

6. Method according to any one of Claims 1 to 5, wherein the transaminase is selected from the group of transaminases and variants thereof which are **characterized in that**, at the position of the amino acid sequence which corresponds to Val224 from the transaminase of *Chromobacterium violaceum* ATCC 12472 (database code NP_901695), it has an amino acid selected from the group consisting of isoleucine, valine, phenylalanine, methionine and leucine, and, at the position of the amino acid sequence which corresponds to Gly230 from the transaminase of *Chromobacterium violaceum* ATCC 12472 (database code NP_901695), has an amino acid other than threonine and preferably an amino acid from the group consisting of serine, cysteine, glycine and alanine.

7. Method according to any one of Claims 1 to 6, wherein step b) and/or step c) is carried out in the presence of an isolated or recombinant alanine dehydrogenase and an inorganic nitrogen source.

8. Method according to any one of Claims 1 to 7, wherein at least one enzyme of the group consisting of NAD(P)⁺-dependent alcohol dehydrogenase, transaminase, monooxygenase and alanine dehydrogenase is recombinant and is provided in the form of a whole cell catalyst which comprises the corresponding enzyme.

9. Method according to Claim 8, wherein all enzymes are provided in the form of one or more as a whole cell catalyst and, preferably, a whole cell catalyst comprises all necessary enzymes.

10. Method according to any one of Claims 1 to 9, wherein in the case of step b), preferably in the case of steps b) and c), an organic cosolvent is present which has a logP of greater than -1.38, preferably 0 to 1.2.

11. Method according to Claim 10, wherein the cosolvent is selected from the group consisting of unsaturated fatty acids, preferably oleic acid.

12. Method according to Claim 10, wherein the cosolvent is a compound of the formula R³-O-(CH₂)ₓ-O-R⁴, wherein R³ and R⁴ are each, and independently of one another, selected from the group consisting of methyl, ethyl, propyl and butyl, and x is 1 to 4, wherein preferably R³ and R⁴ are each methyl and x is 2.

13. Use of a whole cell catalyst comprising an NAD(P)⁺-dependent alcohol dehydrogenase, preferably having at least one zinc atom as cofactor, a transaminase, optionally a monooxygenase, and optionally an alanine dehydrogenase, wherein the enzymes are recombinant enzymes for oxidizing and aminating a primary alcohol of the formula HO-(CH₂)ₓ-R¹, wherein R¹ is selected from the group consisting of -OH, -SH, -NH₂ and -COOR², x is at least 3 and R² is selected from the group consisting of H, alkyl and aryl.

14. Use according to Claim 13, further comprising the presence of organic cosolvent is present which has a logP of greater than -1.38, preferably 0 to 1.2.

15. Use according to Claim 14, wherein the cosolvent is selected from the group consisting of unsaturated fatty acids, preferably oleic acid.

## Revendications

1. Procédé pour l'oxydation d'alcools comprenant les étapes consistant à
a) préparer un alcool primaire de formule
HO-(CH₂)ₓ-R¹
dans laquelle R¹ est choisi dans le groupe comprenant -OH, -SH, -NH₂ et -COOR², x vaut au moins 3 et R² est choisi dans le groupe comprenant H, alkyle et aryle,
b) oxyder l'alcool primaire par mise en contact avec une alcool-déshydrogénase dépendant de NAD(P)⁺ et
c) mettre en contact le produit d'oxydation de l'étape a) avec une transaminase,
l'alcool-déshydrogénase dépendant de NAD(P)⁺ et/ou la transaminase étant une enzyme recombinante ou isolée.

2. Procédé selon la revendication 1, l'étape a) ayant lieu par hydroxylation d'un alcane de formule
H-(CH₂)ₓ-R¹
par une monooxygénase, qui est de préférence recombinante ou isolée.

3. Procédé selon l'une quelconque des revendications 1 à 2, l'alcool-déshydrogénase dépendant de NAD(P)⁺ étant une alcool-déshydrogénase dépendant de NAD(P)⁺ présentant au moins un atome de zinc comme cofacteur.

4. Procédé selon la revendication 3, l'alcool-déshydrogénase étant l'alcool-déshydrogénase de Bacillus stearothermophillus (code de base de données P42328) ou d'une variante de celle-ci.

5. Procédé selon l'une quelconque des revendications 2 à 4, la monooxygénase étant choisie dans le groupe qui comprend AlkBGT de Pseudomonas purida, Cytochrom P450 de Candida tropicalis ou de Cicer arietinum.

6. Procédé selon l'une quelconque des revendications 1 à 5, la transaminase étant choisie dans le groupe des transaminases et de leurs variantes, qui sont **caractérisées en ce qu'**elles présentent, au niveau de la position de la séquence d'acides aminés qui correspond à Val224 de la transaminase de Chromobacterium violaceum ATCC 12472 (code de base de données NP_901695), un acide aminé choisi dans le groupe comprenant l'isoleucine, la valine, la phénylalanine, la méthionine et la leucine et, au niveau de la position de la séquence d'acides aminés qui correspond à Gly230 de la transaminase de Chromobacterium violaceum ATCC 12472 (code de base de données NP_901695), un autre acide aminé que la thréonine et de préférence un acide aminé du groupe comprenant la sérine, la cystéine, la glycine et l'alanine.

7. Procédé selon l'une quelconque des revendications 1 à 6, l'étape b) et/ou l'étape c) étant réalisée(s) en présence d'une alanine-déshydrogénase isolée ou recombinante et d'une source inorganique d'azote.

8. Procédé selon l'une quelconque des revendications 1 à 7, au moins une enzyme du groupe comprenant l'alcool-déshydrogénase dépendant de NAD(P)⁺, la transaminase, la monooxygénase et l'alanine-déshydrogénase étant recombinante et mise à disposition sous forme d'un catalyseur à cellules complètes, qui présente l'enzyme correspondante.

9. Procédé selon la revendication 8, l'ensemble des enzymes étant mis à disposition sous forme d'un ou de plusieurs catalyseurs à cellules complètes et un catalyseur à cellules complètes présentant de préférence toutes les enzymes nécessaires.

10. Procédé selon l'une quelconque des revendications 1 à 9, un cosolvant organique, qui présente un logP supérieur à -1,38, de préférence de 0 à 1,2, étant présent lors de l'étape b), de préférence lors de l'étape b) et de l'étape c).

11. Procédé selon la revendication 10, le cosolvant étant choisi dans le groupe comprenant les acides gras insaturés, de préférence l'acide oléique.

12. Procédé selon la revendication 10, le cosolvant étant un composé de formule R³-O-(CH₂)ₓ-O-R⁴, R³ et R⁴ étant choisis à chaque fois et indépendamment l'un de l'autre dans le groupe comprenant méthyle, éthyle, propyle et butyle et x valant 1 à 4, R³ et R⁴ représentant de préférence à chaque fois méthyle et x valant 2.

13. Utilisation d'un catalyseur à cellules complètes présentant une alcool-déshydrogénase dépendant de NAD(P)⁺, présentant de préférence au moins un atome de zinc comme cofacteur, une transaminase, éventuellement une monooxygénase et éventuellement une alanine-déshydrogénase, les enzymes étant des enzymes recombinantes pour l'oxydation et l'amination d'un alcool primaire de formule HO-(CH₂)ₓ-R¹ dans laquelle R¹ est choisi dans le groupe comprenant -OH, -SH, -NH₂ et -COOR², x vaut au moins 3 et R² est choisi dans le groupe comprenant H, alkyle et aryle.

14. Utilisation selon la revendication 13, comprenant en outre la présence d'un cosolvant organique est présent, qui présente un logP supérieur à -1,38, de préférence de 0 à 1,2.

15. Utilisation selon la revendication 14, le cosolvant étant choisi dans le groupe qui comprend les acides gras insaturés, de préférence l'acide oléique.
